## Europäisches Patentamt
## European Patent Office
## Office européen des brevets

(19)

(11) Veröffentlichungsnummer : **0 006 430**
**B1**

(12) **EUROPÄISCHE PATENTSCHRIFT**

(45) Veröffentlichungstag der Patentschrift :
17.03.82

(21) Anmeldenummer : 79101369.1

(22) Anmeldetag : 04.05.79

(51) Int. Cl.³ : **A 61 F   1/00, A 61 M   3/00**

(54) Tubenartiger Vorratsbehälter für ein medizinisches Spritzgerät zum Mischen und Austragen von pastösen Medien.

(30) Priorität : 30.06.78 DE 7819584 U

(43) Veröffentlichungstag der Anmeldung :
09.01.80 (Patentblatt 80/01)

(45) Bekanntmachung des Hinweises auf die Patenterteilung : 17.03.82 Patentblatt 82/11

(84) Benannte Vertragsstaaten :
AT BE CH DE FR GB IT LU NL SE

(56) Entgegenhaltungen :
CH - A - 248 985
DE - A - 2 004 465
DE - A - 2 814 353
FR - A - 957 829
FR - A - 1 161 417
US - A - 3 417 971

(73) Patentinhaber : Howmedica International, Inc. Zweigniederlassung Kiel
Professor-Küntscher-Strasse 1-5
D-2301 Schönkirchen üb. Kiel (DE)

(72) Erfinder : Richter, Karl M, Dipl.-Ing
Haferkamp 14
D-2304 Wendtorf (DE)
Erfinder : Harder, Hans Erich
Mecklenburger Strasse 32
D-2301 Probsteierhagen (DE)
Erfinder : Behrens, Klaus, Ing.grad
Am Sportplatz 8
D-2351 Rickling (DE)

(74) Vertreter : Hauck, Hans, Dipl.-Ing. et al
Patentanwälte Dipl.-Ing.H.Hauck,
Dipl.-Phys.W.Schmitz, Dipl.-Ing.E.Graalfs,
Dipl.-Ing.W.Wehnert, Dipl.-Phys.W.Carstens
Dr.-Ing.W.Döring Neuer Wall 41
D-2000 Hamburg 36 (DE)

Imprimerie Jouve, 18, rue St-Denis, 75001 Paris, France

# Tubenartiger Vorratsbehälter für ein medizinisches Spritzgerät zum Mischen und Austragen von pastösen Medien

Die Erfindung bezieht sich auf einen tubenartigen Vorratsbehälter für ein medizinisches Spritzgerät zum Mischen und Austragen von pastösen Medien, insbesondere Knochenzement, mit Hilfe eines, eine Vorschubstange enthaltenden Handgerätes, mit einem beidendig offenen zylindrischen, in das Handgerät einlegbaren Rohr, einem stopfenartigen Kolben, der dichtend und gleitend im Rohr verschiebbar und von der Vorschubstange betätigbar ist, und einem mit dem vorderen Ende des Rohres verbindbaren Spritzrohr.

Ein derartiger Vorratsbehälter ist bereits in der nachveröffentlichten DE-A-2814353 vorgeschlagen worden. Zum Mischen wird das beidendig offene Rohr in das Handgerät eingelegt, wodurch der Mischvorgang verhältnismäßig umständlich wird, zumal ein lagesicheres Aufstellen des Handgerätes ohne zusätzliche Vorkehrungen nicht möglich ist. Auch besteht Gefahr, daß das Handgerät beim Befüllen verschmutzt wird. Nachteilig ist ferner, daß ein offenes Ende des Rohres mit einem geeigneten Dichtverschluß während des Mischens verschlossen werden muß, wobei der Dichtverschluß nach dem Aufsetzen eines Spritzabschnittes nicht mehr benötigt wird.

Es ist ferner eine Vorrichtung zum Zubereiten und Verarbeiten von dentalmedizinischen Abformpasten bekannt, bei der die Abformpaste in einem einen Kolben enthaltenden Behälter hergestellt wird. Ist die gewünschte Mischung fertig, wird eine Spritze auf die offene Seite des Behälters aufgedrückt, so daß der auf einer Scheibe sich abstützende Kolben nach oben fährt und die Paste in die Spritze hineindrückt (DE-A-2 004 465).

Schließlich ist bekannt, einen Untersatz für Tuben zur Aufnahme von Zahnpasta, kosmetischen Cremes oder anderen Pasten vorzusehen, in dem die offenen Tuben dichtend augestellt werden können (CH-A-248 985). Mit dem bekannten Untersatz kann das lästige Verschließen von Tuben mit einer Verschlußkappe, das häufig vergessen wird, vermieden werden.

Der Erfindung liegt die Aufgabe zugrunde, einen tubenartigen Vorratsbehälter für ein medizinisches Spritzgerät für pastöse Medien zu schaffen, welcher auf besonders einfache Weise das Mischen und Austragen des Mediums ermöglicht.

Diese Aufgabe wird erfindungsgemäß dadurch gelöst, daß das Spritzrohr zweiteilig ist mit einem auf die Außenseite des Rohres aufschraubbaren, erweiterten ersten Abschnitt und einem schlauchartigen zweiten Abschnitt, der klemmend auf einem Ansatz des ersten Abschnitts befestigbar ist, und ein an beiden Enden offener Trichter mit einem zylindrischen Abschnitt vorgesehen ist, wobei der zylindrische Abschnitt auf die Außenseite der Enden des Rohres aufsteckbar ist und im Inneren einen Anschlag aufweist, der mit der Stirnseite der Rohrenden

zusammenwirkt.

Häufig sind derartige tubenartige Vorratsbehälter in vorteilhafter Weise sogenannte Wegwerfbehälter oder Einmalbehälter und zu diesem Zwecke aus einem geeigneten Kunststoffmaterial hergestellt. Für den erfindungsgemäßen tubenartigen Vorratsbehälter gilt dies im besonderen Maße. Bei der Erfindung besitzt der tubenartige Vorratsbehälter ein beidendig offenes Rohr, das mit Hilfe eines geeignet ausgebildeten Trichters besonders einfach mit den zu mischenden Bestandteilen gefüllt und zum Mischen anschließend auf einfache Weise aufgestellt werden kann. Auch zur Befestigung des Spritzrohres, das einfach auf die Außenseite des Behälterrohres aufgeschraubt wird, kann der Trichter als Standuntersatz wertvolle Hilfe leisten. Das Spritzrohr ist aus Fertigungsgründen zweiteilig ausgebildet mit einem auf das Rohr aufschraubbaren erweiterten Abschnitt und einem schlauchartigen Abschnitt, der klemmend mit dem erweiterten Abschnitt verbindbar ist.

Der schlauchartige Abschnitt ist vorzugsweise aus einem Material, das mit Hilfe eines Skalpells oder eines ähnlich scharfen Gegenstandes auf die gewünschte Länge verkürzt werden kann.

Der Anschlag innerhalb des Trichters verhindert darüber hinaus, daß beim Einfüllen der pastösen Medien der kolbenförmige Stopfen herausfällt.

Zur Erzielung einer günstigen Strömungseigenschaft ist gemäß einer Ausgestaltung der Erfindung der erste Abschnitt des Spritzrohres konisch erweitert. Das Gewinde des ersten Abschnittes ist gemäß einer weiteren Ausgestaltung der Erfindung ein Schnellverschlußgewinde. Dadurch wird die Zeit, innerhalb der der erfindungsgemäße Vorratsbehälter gebrauchsfertig erstellt wird, verringert.

Der Anschlag innerhalb des Trichters läßt sich auf verschiedene Weise ausbilden. Eine bevorzugte Ausgestaltung der Erfindung sieht vor, daß der Anschlag des Trichters von einem ringförmigen Bund vor dem Übergang zum konischen Teil gebildet ist.

Für den Kolben sieht eine Ausgestaltung der Erfindung vor, daß er einen vorderen Plattenabschnitt und einen mit ringförmig umlaufenden flexiblen Rippen geformten hinteren Abschnitt aufweist, wobei der Durchmesser des zylindrischen Plattenabschnitts dem Innendurchmesser des Rohres entspricht und der Durchmesser der Rippen etwas größer ist als der Innendurchmesser des Rohres.

Bei dem Einsetzen des Kolbens in das Rohr werden die flexiblen Rippen etwas nach hinten und einwärts verformt, so daß der Kolben formschlüssig im Inneren der Tube gehalten wird und sich darin auch selbst hält. In diesem Zusammenhang sieht eine weitere Ausgestaltung der Erfindung vor, daß die Rippen im Querschnitt sägezahnförmige Flanken aufweisen, wobei in Vorschubrichtung gesehen die vordere Flanke einen kleineren Winkel zur Längsachse einschließt als die hintere Flanke und der Scheitel der Rippen als zum zylindrischen Abschnitt parallele Ringfläche geformt ist. Es hat sich gezeigt, daß insbesondere diese Form des Kolbens eine besonders leichte Betätigung und gleichzeitig wirksame Abdichtung im Rohr ermöglicht.

Ein Ausführungsbeispiel der Erfindung wird nachfolgend anhand von Zeichnungen näher beschrieben.

Fig. 1 zeigt einen Schnitt durch einen Vorratsbehälter nach der Erfindung.

Fig. 2 zeigt einen Schnitt durch einen Teil des Kolbens des Vorratsbehälters nach Fig. 1.

Fig. 3 zeigt einen Schnitt durch einen anderen Teil des Vorratsbehälters nach Fig. 1.

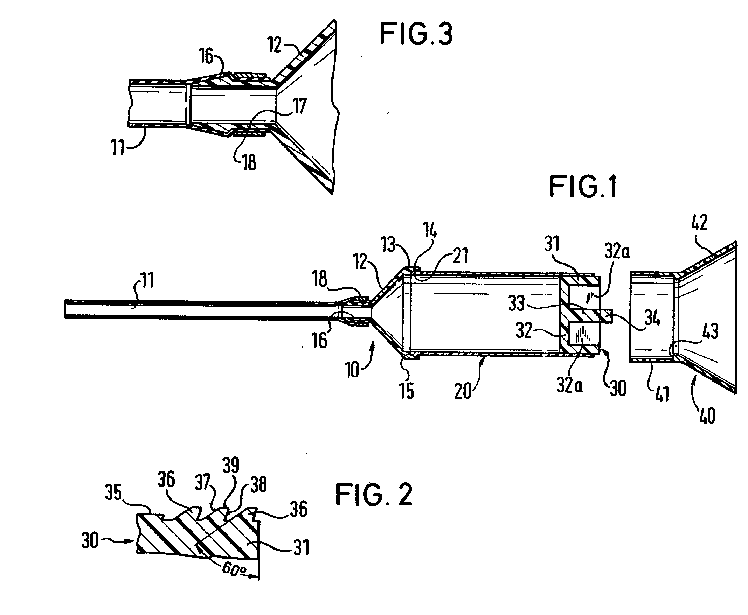

Der in Fig. 1 gezeigte Vorratsbehälter besteht aus vier zusammensetzbaren Teilen, nämlich einem zweiteiligen Spritzrohr 10, einer zylindrischen Tube 20, einem Kolben 30 und einem Trichter 40. Die Tube 20 ist ein verhältnismäßig dünnwandiger Zylinder mit durchgehender Innenbohrung, so daß er an beiden Enden offen ist. Am vorderen Ende ist auf der Außenseite ein Schnellverschlußgewinde 21 geformt.

Aus fertigungstechnischen Gründen besteht das Spritzrohr 10 aus zwei Teilen, nämlich einem verhältnismäßig langen Rohrabschnitt 11 auf mit verhältnismäßig kleinem Innendurchmesser, der aus einem serienmäßig hergestellten Schlauch gewonnen ist, der in gewünschter Länge abgeschnitten ist. Seine Befestigung wird anhand von Fig. 3 näher beschrieben. Am hinteren Ende ist das Spritzrohr 10 mit einem konischen Abschnitt 12 verbunden, der mit einem zylindrischen Abschnitt 13 die Außenseite der Tube 20 übergreift. Ein Innenschnellverschlußgewinde 14 des konischen Abschnitts 12 wirkt mit dem Gewinde 21 der Tube 20 zusammen. Im Innern des zylindrischen Abschnitts 13 ist eine Schulter 15 geformt, gegen die die Stirnseite der Tube 20 anliegt, wobei der Innendurchmesser des zylindrischen Abschnitts 13 mit dem der Tube 20 gleich ist, so daß ein gleichmäßiger Übergang zwischen konischem Abschnitt 12 und Tube 20 geschaffen ist. Ein Anschluß 16 sitzt am engeren Teil des konischen Abschnitts 12, über den der Schlauch 11 gezogen ist (Fig. 3).

Der Kolben 30 besitzt einen Zylinderring, der am vorderen Ende durch eine massive Scheibe 32 abgeschlossen ist. Im Innern des Ringes 31 sind sich senkrecht kreuzende Stege 32a und 33 angeordnet und mit dem scheibenförmigen Teil 32 verbunden. Vom Kreuzungspunkt der Stege 32a, 33 erstreckt sich nach hinten ein Zapfen 34, mit dem die nichtgezeigte Schubstange eines Einspritzgerätes zusammenwirkt.

Der Kolbenring 31 besitzt, wie aus Fig. 2 hervorgeht, einen Abschnitt 35, dessen Außenseite glatt zylindrisch geformt ist. Er erstreckt sich über etwa 2/3 der Länge des Kolbens. Daran schliessen sich nach hinten drei ringförmig umlaufende Rippen 36 an, die im Querschnitt sägezahnartig ausgebildet sind, wobei deren vordere Flanke 37 einen geringeren Winkel zur Längsachse des Kolbens 30 anschließen als die hintere Flanke 38. Außen besitzen die Rippen 36 einen glatten ringförmigen Abschnitt 39, der parallel und koaxial zum zylindrischen Abschnitt 35 verläuft. Der Außendurchmesser des zylindrischen Abschnitts 35 entspricht dem Innendurchmesser der Tube 20. Der Außendurchmesser der Rippen 36 ist etwas größer als der Innendurchmesser der Tube, so daß die Rippen 36 beim Einsetzen des Kolbens 30 elastisch nach hinten und radial einwärts verformt werden.

Der Trichter 40 weist einen zylindrischen Teil 41 auf, dessen Innendurchmesser etwa dem Außendurchmesser der Tube 20 entspricht Daran schließt sich ein konischer Teil 42 an. Am Übergang zwischen dem zylindrischen Teil 41 und dem konischen Teil 42 ist ein ringförmiger Bund 43 geformt, der als Anschlag wirkt, wenn der Trichter 40 über eines der beiden Enden der Tube 20 geschoben wird. Wird der Trichter über das vordere Ende geschoben, dient er als Einfülltrichter. Wird er auf das hintere Ende geschoben, dient er als Ständer und verhindert gleichzeitig, daß der Kolben 30 aus der Tube nach unten herausfällt.

Die Teile 10, 20, 30 und 40 können aus einem geeigneten Kunststoffmaterial hergestellt werden. Sie werden vor dem Gebrauch zweckmäßigerweise einzeln in einer sterilen Verpackung untergebracht. Das Injektionsrohr 11 hat eine verhältnismäßig große Länge, beispielsweise bis zu 36 cm. Sein Material ist derart gewählt, daß eine Verkürzung mit einem Skalpell oder scharfen Messer auf jede beliebige Länge möglich ist. Auf diese Weise können auch Langschaftprothesen versorgt werden.

Die Befestigung des Schlauchstücks 11 am Ansatz 16 ist in Fig. 3 zu erkennen. Der Ansatz 16 verjüngt sich zum vorderen Ende konisch und weist im hinteren Bereich eine Ringnut 17 auf zur Befestigung des Schlauchstücks 11 mittels eines Klemmrings 18 aus rostfreiem Stahl. Das Schlauchstück 11 kann unter Umständen auch ohne einen derartigen Klemmring 18 ausreichend fest auf dem Anschluß 16 sitzen. Üblicherweise sind Schlauchstück 11 und Abschnitt 12 herstellerseitig schon fertig montiert.

## Ansprüche

1. Tubenartiger Vorratsbehälter für ein medizinisches Spritzgerät zum Mischen und Austragen von pastösen Medien, insbesondere Knochenzement, mit Hilfe eines, eine Vorschubstange enthaltenden Handgerätes, mit einem beidendig offenen zylindrischen, in das Handgerät einlegbaren Rohr (20), einem stopfenartigen Kolben (30), der dichtend und gleitend im Rohr (20) verschiebbar und von der Vorschubstange betätigbar ist, und einem mit dem vorderen Ende des Rohres (20) verbindbaren Spritzrohr (10), dadurch gekennzeichnet, daß das Spritzrohr (10) zweitel-

lig ist mit einem auf die Außenseite des Rohres (20) aufschraubbaren, erweiterten ersten Abschnitt (12) und einem schlauchartigen zweiten Abschnitt (11), der klemmend auf einem Ansatz (16) des ersten Abschnitts (12) befestigbar ist, und ein an beiden Enden offener Trichter (40) mit einem zylindrischen Abschnitt (41) vorgesehen ist, wobei der zylindrische Abschnitt (41) auf die Außenseiten der Enden des Rohres (20) aufsteckbar ist und im Inneren einen Anschlag (43) aufweist, der mit der Stirnseite der Rohrenden zusammenwirkt.

2. Vorratsbehälter nach Anspruch 1, dadurch gekennzeichnet, daß der erste Abschnitt (12) des Spritzrohrs (10) sich konisch erweitert.

3. Vorratsbehälter nach Anspruch 1 oder 2, dadurch gekennzeichnet, daß das Gewinde des ersten Abschnitts (12) des Spritzrohrs (10) ein Schnellverschlußgewinde ist.

4. Vorratsbehälter nach einem der Ansprüche 1 bis 3, dadurch gekennzeichnet, daß der Anschlag (43) des Trichters (40) von einem ringförmigen Bund vor dem Übergang zum konischen Teil (42) gebildet ist.

5. Vorratsbehälter nach einem der Ansprüche 1 bis 4, dadurch gekennzeichnet, daß der Kolben (30) einen vorderen Plattenabschnitt (35) und einen mit ringförmig umlaufenden flexiblen Rippen (36) geformten hinteren Abschnitt aufweist, wobei der Durchmesser des zylindrischen Plattenabschnitts (35) dem Innendurchmesser des Rohres (20) entspricht und der Durchmesser der Rippen (36) etwas größer ist als der Innendurchmesser des Rohres (20).

6. Vorratsbehälter nach Anspruch 5, dadurch gekennzeichnet, daß die Rippen (36) im Querschnitt sägezahnförmige Flanken aufweisen, wobei in Vorschubrichtung gesehen die vordere Flanke (37) einen kleineren Winkel zur Längsachse einschließt als die hintere Flanke (38) und der Scheitel der Rippen (36) als zum zylindrischen Abschnitt (35) parallele Ringfläche (39) geformt ist.

**Claims**

1. A tube-shaped supply container for a medical injection device for mixing and discharging pasty media, especially bone cement, with the aid of a manual apparatus comprising a feeding rod, a cylindrical tube (20) open at both ends adapted to be placed into the manual apparatus, a stopper-like piston (30) sealingly and slidably displaceable in the tube (20) and operable by the feeding rod, and an injection tube (10) adapted to be connected to the forward end of the tube (20), characterized in that the injection tube (10) is bipartite having an enlarged first portion (12) adapted to be screwed onto the outer surface of the tube (20) and a hose-like second portion (11) adapted to be fastened in clamping engagement on an attachment (16) of the first portion (12), and in that there is provided a funnel (40) which is open at both ends and comprises a cylindrical portion (41), said cylindrical portion (41) being adapted to be slipped onto the outer surfaces of the ends of the tube (20) and having an abutment (43) in the interior thereof which cooperates with the end face of the tube ends.

2. A supply container according to claim 1, characterized in that the first portion (12) of the injection tube (10) enlarges conically.

3. A supply container according to claim 1 or 2, characterized in that the thread of the first portion (12) of the injection tube (10) is a quick-closing thread.

4. A supply container according to any one of the claims 1 to 3, characterized in that the abutment (43) of the funnel (40) is formed by an annular flange in front of the transition to form the conical portion (42).

5. A supply container according to any one of the claims 1 to 4, characterized in that the piston (30) comprises a forward plate section (35) and a rearward section formed with circumferentially extending annular ribs (36), the diameter of the cylindrical plate section (35) corresponding to the inner diameter of the tube (20) and the diameter of the ribs (36) being slightly greater than the inner diameter of the tube (20).

6. A supply container according to claim 5, characterized in that the ribs (36) are provided with flanks having a saw-tooth shaped cross sectional area, with the forward flank (37) in the direction of feed including a smaller angle with the longitudinal axis than the rearward flank (38) and with the apex of the ribs (36) being formed as an annular surface (39) in parallel with the cylindrical portion (35).

**Revendications**

1. Réservoir en forme de tube pour instrument médical d'injection, servant à mélanger et à décharger des produits pâteux, en particulier du ciment pour os, à l'aide d'un instrument à main muni d'un poussoir, réservoir comportant un tube cylindrique (20), ouvert aux deux bouts, et pouvant être introduit dans l'instrument à main, un piston en forme de bouchon (30), qui peut coulisser de façon étanche en glissant dans le tube (20), et qui peut être actionné par le poussoir, et un tube d'injection (10), pouvant être relié à l'extrémité antérieure du tube (20), et caractérisé par le fait que le tube d'injection (10) est en deux parties, notamment un premier élément élargi (12), qui peut être vissé sur l'extérieur du tube (20), et un deuxième élément en forme de tube (11), qui peut être fixé avec serrage sur un appendice (16) du premier élément (12), et qu'un entonnoir (40), ouvert à ses deux extrémités, comporte un élément cylindrique (41), qui peut être engagé par l'extérieur sur les bouts du tube (20), et qui présente intérieurement une butée (43), coopérant avec la face frontale de l'un des bouts du tube.

2. Réservoir selon la revendication 1, caractérisé par le fait que le premier élément (12) du tube d'injection (10) va en s'élargissant de façon conique.

3. Réservoir selon l'une des revendications 1 et 2, caractérisé par le fait que le filetage du premier élément (12) du tube d'injection (10) est un filetage pour fermeture rapide.

4. Réservoir selon l'une quelconque des revendications 1 à 3, caractérisé en ce que la butée (43) de l'entonnoir (40) est formée par un collet annulaire précédant le raccordement avec la partie conique (42).

5. Réservoir selon l'une quelconque des revendications 1 à 4, caractérisé en ce que le piston (30) présente un élément antérieur cylindrique (35), et un élément postérieur muni de nervures annulaires flexibles (36), le diamètre de l'élément cylindrique (35) correspondant au diamètre intérieur du tube (20), et le diamètre des nervures (36) étant un peu supérieur au diamètre intérieur du tube (20).

6. Réservoir selon la revendication 5, caractérisé en ce que les nervures (36) présentent en section des flancs en dents de scie, le flanc avant (37) dans le sens de l'avancement, faisant un plus petit angle avec l'axe longitudinal, que le flanc arrière (38), et le sommet des nervures (36) ayant la forme d'une surface annulaire (39), parallèle à l'élément cylindrique (35).

## FIG.3

16  12
11  17
18

## FIG.1

13  14
12  21  31  32a  42
18
11
16  33  34
10  32  43
15  20  32a  30  41  40

## FIG. 2

39
36  37  38
35  36
30  31
60°

0 006 430